# EUROPÄISCHE PATENTSCHRIFT

(11) **EP 1 699 386 B1**
(45) Veröffentlichungstag und Bekanntmachung des Hinweises auf die Patenterteilung: **20.01.2010**
(21) Anmeldenummer: 04803788.1
(22) Anmeldetag: 13.12.2004
(51) Int. Cl.: A61F 2/34

(54) **GELENKPFANNE FÜR EINE HÜFTENDOPROTHESE**
JOINT SOCKET FOR A HIP ENDOPROSTHESIS
COTYLE POUR UNE ENDOPROTHESE DE LA HANCHE

(30) Priorität: 22.12.2003 DE 10360390
(43) Veröffentlichungstag der Anmeldung: 13.09.2006
(73) Patentinhaber: Smith & Nephew Orthopaedics AG, 6343 Rotkreuz (CH)
(72) Erfinder: IMHOF, Martin, 6343 Rotkreuz (CH)
(74) Vertreter: Popp, Eugen
(86) Internationale Anmeldenummer: PCT/EP2004/014151
(87) Internationale Veröffentlichungsnummer: WO 2005/063148

(56) Entgegenhaltungen:
- EP-A- 0 655 230
- EP-A- 0 694 294
- DE-A1- 4 335 931
- DE-A1- 4 428 290

## Beschreibung

Die Erfindung betrifft eine Gelenkpfanne für eine Hüftendoprothese gemäß dem Oberbegriff des Anspruchs 1, wie sie beispielsweise aus der DE-A-4428290 bekannt ist.

Bei Hüfttotalendoprothesen wird in das Femur ein Prothesenschaft mit einem Gelenkkopf eingesetzt. In den Beckenknochen wird eine Gelenkpfanne implantiert, die als Lagerschale für den Gelenkkopf dient. Es ist bekannt, die Gelenkpfanne aus einer Pfannenschale und einem Pfanneneinsatz aufzubauen. Die Pfannenschale kann in Bezug auf die Implantation in den Beckenknochen optimiert werden, während der Pfanneneinsatz in Bezug auf die Lagereigenschaften für den Gelenkkopf optimiert werden kann. Dabei wird die Pfannenschale so gestaltet und so in dem Beckenknochen positioniert, dass ein möglichst stabiles Einwachsen der Pfannenschale in den Beckenknochen möglich ist. Der Pfanneneinsatz kann in der Pfannenschale so ausgerichtet werden, dass der Gelenkkopf mit möglichst korrekter orthopädischer Lage des Prothesenschaftes und damit des Femurs des Patienten aufgenommen wird.

Aus der EP 0 663 193 A1 ist eine Gelenkpfanne bekannt, bei welcher der Pfanneneinsatz eine sphärische Außenfläche aufweist und mit dieser sphärischen Außenfläche in einem sphärischen Aufnahmeraum mit gleichem Kugelradius der Pfannenschale sitzt. Beim Einsetzen des Pfanneneinsatzes in die Pfannenschale kann der Pfanneneinsatz daher beliebig um seine Rotationsachse gedreht und mit seiner Rotationsachse beliebig gegenüber der Rotationsachse des Aufnahmeraumes gekippt werden. Dadurch ist es möglich, die Pfannenschale in dem Beckenknochen entsprechend der Knochenstruktur zu positionieren. Der Pfanneneinsatz kann entsprechend der orthopädischen Lage des in das Femur eingesetzten Prothesenschaftes ausgerichtet werden. Um den Pfanneneinsatz in seiner Lage in der Pfannenschale zu fixieren, weist die sphärische Innenfläche des Aufnahmeraumes der Pfannenschale spitz vorstehende Zähne auf, die in die Außenfläche des Pfanneneinsatzes eingreifen. Da die Zähne in die Außenfläche des Pfanneneinsatzes eindringen müssen, bestehen Beschränkungen in Bezug auf die Wahl des Materials des Pfanneneinsatzes. Das Einpressen des Pfanneneinsatzes auf die Zähne der Pfannenschale erschwert das exakt positionierte Einsetzen des Pfanneneinsatzes.

Der Erfindung liegt die Aufgabe zugrunde, eine Gelenkpfanne für eine Hüftendoprothese zur Verfügung zu stellen, die eine freie Ausrichtung des Pfanneneinsatzes in Bezug auf die Pfannenschale mit hoher Präzision und feinfühlig zulässt.

Diese Aufgabe wird erfindungsgemäß gelöst durch eine Gelenkpfanne mit den Merkmalen des Anspruchs 1.

Vorteilhafte Ausführungen der Erfindung sind in den Unteransprüchen angegeben.

Erfindungsgemäß weist die Gelenkpfanne einen Pfanneneinsatz auf, der aufgrund seiner sphärischen Außenfläche eine freie Drehbarkeit und Verkippbarkeit in der Pfannenschale zulässt. Die Pfannenschale kann daher entsprechend der Anatomie und der Struktur des Beckenknochens implantiert werden, so dass optimale Einwachsbedingungen erzielt werden können. Der Pfanneneinsatz kann in der Pfannenschale so gedreht und mit seiner Rotationsachse gegen die Rotationsachse der Pfannenschale gekippt werden, dass die Rotationsachse des Pfanneneinsatzes mit der Achse des Schenkelhalses des Prothesenschaftes fluchtet, wenn das Femur mit dem eingesetzten Prothesenschaft in der orthopädisch optimalen Position angeordnet ist. Die sphärische Außenfläche des Pfanneneinsatzes berührt die Innenfläche des Aufnahmeraumes längs einer Umfangslinie, die zu der Rotationsachse des Aufnahmeraumes konzentrisch verläuft. Aufgrund dieser linienförmigen Berührung ist ein leichtes Drehen und Kippen des Pfanneneinsatzes in dem Aufnahmeraum möglich, um den Pfanneneinsatz optimal in seiner Lage auszurichten. Sobald der Pfanneneinsatz ausgerichtet ist, genügt ein leichter Druck, um den Pfanneneinsatz in den sich verengenden Aufnahmeraum einzupressen, worauf der Pfanneneinsatz in dem Aufnahmeraum selbsthemmend geklemmt wird. Die selbsthemmende Klemmung bewirkt eine Fixierung des Pfanneneinsatzes in der Pfannenschale mit hoher Stabilität. Eine Belastung des Gelenkes bewirkt dabei ein zusätzliches Einpressen des Pfanneneinsatzes in die Pfannenschale, so dass die Fixierung der Pfannenschale zusätzlich verstärkt wird.

Da sich die Fixierung des optimal ausgerichteten Pfanneneinsatzes durch einfaches Eindrücken in den Aufnahmeraum ergibt, ist diese Fixierung einfach durchführbar und erfordert keine zusätzlichen Instrumente oder zusätzliche Befestigungsmittel. Die selbsthemmende Klemmung stellt sich bei einem minimalen Verschiebungsweg des Pfanneneinsatzes in dem Aufnahmeraum ein, so dass bei dem Fixieren des Pfanneneinsatzes keine unbeabsichtigte Dejustage der Ausrichtung des Pfanneneinsatzes auftreten kann.

Bei implantierter Prothese kann in ungünstigen Fällen der Schenkelhals des Prothesenschaftes an dem Rand der Gelenkpfanne anschlagen (sog. Impingement). Dadurch übt der Prothesenschaft ein Hebelmoment auf die Gelenkpfanne aus. Bei herkömmlichen Gelenkpfannen, bei welchen der Pfanneneinsatz formschlüssig in der Pfannenschale gehalten wird, kann dieses Hebelmoment dazu führen, dass die gesamte Gelenkpfanne aus dem Beckenknochen gehebelt oder zumindest in dem Beckenknochen gelockert wird. Da erfindungsgemäß der Pfanneneinsatz nur in den Aufnahmeraum der Pfannenschale eingepresst ist, bewirkt ein solches Hebelmoment bei der erfindungsgemäßen Gelenkpfanne im ungünstigen Falle nur eine Lockerung des Pfanneneinsatzes in der Pfannenschale. Bei einer anschließenden regulären Belastung des Gelenkes wird der Pfanneneinsatz wieder in den Aufnahmeraum der Pfannenschale eingepresst und erneut festgeklemmt und fixiert.

In einer bevorzugten Ausführung ist die Innenfläche des Aufnahmeraumes der Pfannenschale zumindest in dem Bereich der Berührungslinie als sich gegen den Pol des Aufnahmeraumes verengender Konus ausgebildet. Dadurch ist eine einfache Herstellung möglich. Die Konusfläche gewährleistet außerdem eine besonders wirksame Selbsthemmung. Als Konuswinkel, d. h. als Winkel zwischen der Konusmittelachse und der Konusmantellinie wird der der Materialpaarung von Pfannenschale und Pfanneneinsatz entsprechende Selbsthemmungswinkel gewählt. Üblicherweise liegt dieser Konuswinkel je nach Materialpaarung bei etwa 4° bis 10°.

Um eine zuverlässige Klemmung des Pfanneneinsatzes in der Pfannenschale zu bewirken, werden der Pfanneneinsatz und die Pfannenschale aus einem harten Werkstoff gefertigt. Die Pfannenschale wird vorzugsweise aus einem biokompatiblen Metall hergestellt, z. B. einer Titan-Legierung. Für den Pfanneneinsatz kann ein Werkstoff entsprechend der Gleitpaarung von Pfannenschale und Gelenkkopf gewählt werden, z. B. ein metallischer oder keramischer Werkstoff oder ein Kunststoff.

Im folgenden wird die Erfindung anhand eines in der Zeichnung dargestellten Ausführungsbeispiels näher erläutert. Es zeigen:
- Figur 1: eine Hüfttotalendoprothese und
- Figur 2: einen Axialschnitt durch die Gelenkpfanne dieser Prothese.

Die Hüfttotalendoprothese besteht aus einer in den Beckenknochen 10 implantierbaren Gelenkpfanne und einem Prothesenschaft 12, der in das Femur 40 eingesetzt wird. Der Prothesenschaft 12 weist einen Schenkelhals 14 auf, auf welchem ein Gelenkkopf 16 sitzt, der in der Gelenkpfanne gelagert wird.

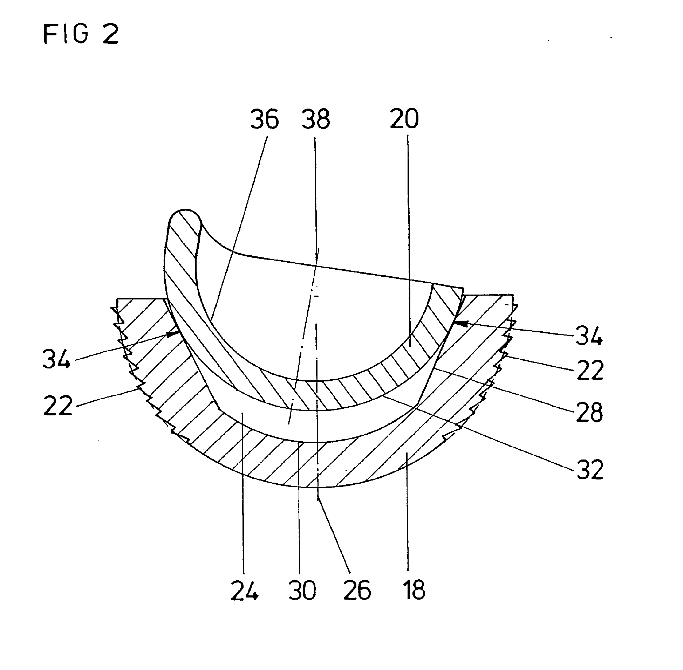

Die in Figur 2 gesondert dargestellte Gelenkpfanne besteht aus einer Pfannenschale 18 und einem Pfanneneinsatz 20. Die Pfannenschale 18 wird in einer an sich bekannten Weise in den Beckenknochen 10 eingesetzt. Hierzu kann die Pfannenschale 18 mittels zusätzlicher Schrauben in dem Beckenknochen 10 befestigt werden. Die Pfannenschale 18 kann als Schraubpfanne ausgebildet sein, die an ihrer Außenfläche ein Gewinde aufweist, als Einpresspfanne, die an ihrer Außenfläche mit einer geeigneten Struktur 22 ausgebildet ist, wie dies in Figur 2 beispielhaft angedeutet ist, oder als Reoperationspfanne, wie dies z.B. im EP 0 663 193 A1 beschrieben ist.

Die im wesentlichen halbkugelförmige Pfannenschale 18 ist durch einen Aufnahmeraum 24 ausgehöhlt, der sich gegen die Äquatorebene hin öffnet. Der Aufnahmeraum 24 ist in Bezug auf die Mittelachse 26 der Pfannenschale 18 rotationssymmetrisch. Der Aufnahmeraum 24 weist eine Innenfläche 28 in Form eines geraden Kreiskegels auf, die sich von der in der Äquatorebene liegenden Öffnung gegen den Pol der Pfannenschale 18 hin verengt. Der polseitige Grund 30 des Aufnahmeraumes 24 ist abgeflacht. Der Kegelwinkel der konischen Innenfläche 28, d. h. der zwischen der Rotationsachse 26 und der Mantellinie der Innenfläche 28 eingeschlossene Winkel wird je nach der Materialpaarung von Pfannenschale 18 und Pfanneneinsatz 20 so gewählt, dass sich eine Selbsthemmung ergibt. Vorzugsweise liegt dieser Winkel bei etwa 4° bis 10°. Bei einer metallischen Pfannenschale 18 ergibt sich z.B. für einen metallischen Pfanneneinsatz 20 ein selbsthemmender Kegelwinkel von ca.4,5° und für einen keramischen Pfanneneinsatz 20 ein selbsthemmender Kegelwinkel von ca. 9,5°.

Der Pfanneneinsatz 20 ist ebenfalls im wesentlichen halbkugelförmig ausgebildet. Die Außenfläche 32 des Pfanneneinsatzes 20 ist zumindest in dem Bereich, in welchem diese Außenfläche 32 mit der Innenfläche 28 des Aufnahmeraumes 24 in Berührung kommt, sphärisch ausgebildet. Der Durchmesser der Außenfläche 32 entspricht dem Durchmesser der Innenfläche 28 in einer Berührungslinie 34, die etwas von der äquatorialen Austrittsebene beabstandet (ca. 5 mm bis 15 mm) innerhalb des Aufnahmeraumes 24 konzentrisch zur Rotationsachse 26 verläuft.

Der Pfanneneinsatz 20 weist eine ausgehöhlte sphärische Lagerfläche 36 auf, die zur Aufnahme und Lagerung des Gelenkkopfes 16 dient. Die sphärische Außenfläche 32 und die sphärische Lagerfläche 36 sind zu einer Rotationsachse 38 des Pfanneneinsatzes 20 rotationssymmetrisch.

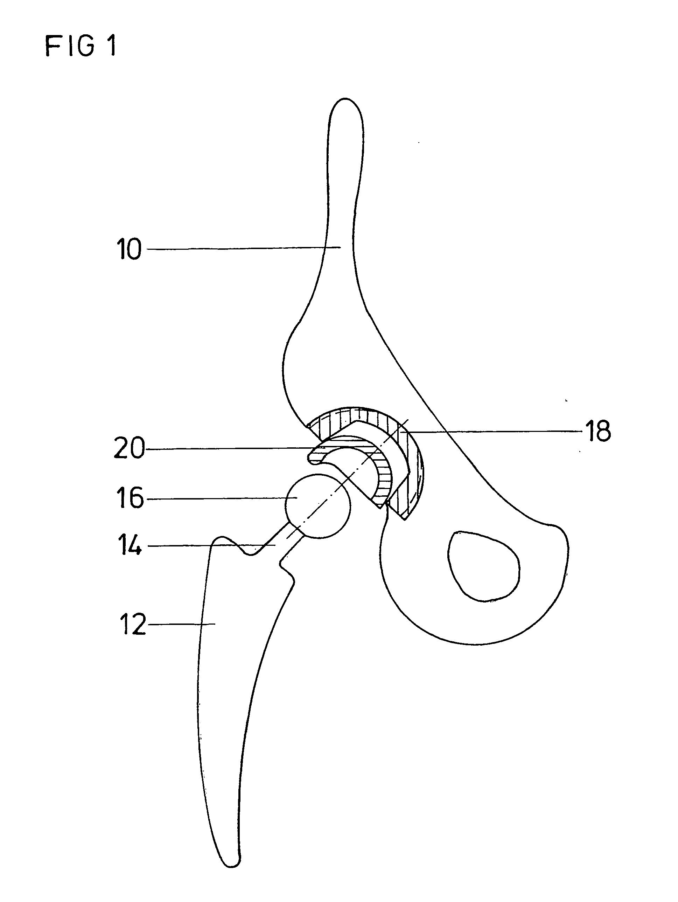

Die Pfannenschale 18 wird in den Beckenknochen 10 eingesetzt, wie dies in Figur 1 gezeigt ist, wobei die Anordnung der Pfannenschale 18 in dem Beckenknochen 10 entsprechend der Anatomie und der Struktur des Beckenknochens 10 gewählt wird. Dann wird der Pfanneneinsatz 20 lose in den Aufnahmeraum 24 der Pfannenschale 18 eingesetzt. Der Pfanneneinsatz 20 kommt mit seiner Außenfläche 32 längs der Berührungslinie 34 in Berührung mit der konischen Innenfläche 28 des Aufnahmeraumes 24. Dabei lässt sich der Pfanneneinsatz 20 beliebig um seine Rotationsachse 38 drehen und die Rotationsachse 38 des Pfanneneinsatzes 20 lässt sich beliebig gegenüber der Rotationsachse 26 der Pfannenschale 18 kippen.

Der Prothesenschaft 12 wird in den freigelegten Markhohlraum des Femurs 40 eingeschlagen, wobei sich der Prothesenschaft 12 in seiner Drehstellung eventuell geringfügig der Knochenstruktur des Femurs anpasst. Dadurch wird die Lage und Orientierung des Schenkelhalses 14 mit dem Gelenkkopf 16 in Bezug auf das Femur festgelegt. Nun wird der Gelenkkopf 16 in die Lagerfläche 36 des Pfanneneinsatzes 20 eingesetzt und das Femur 40 mit dem Prothesenschaft 12 in die orthopädisch optimale Position gebracht. Der Pfanneneinsatz 20 kann dabei entsprechend dieser Positionierung ausgerichtet werden. Sobald der Pfanneneinsatz 20 optimal ausgerichtet ist, wird der Pfanneneinsatz 20 axial in den Aufnahmeraum 24 eingedrückt, so dass er selbsthemmend in dieser Ausrichtungsstellung geklemmt wird.

### Bezugszeichenliste

- 10: Beckenknochen
- 12: Prothesenschaft
- 14: Schenkelhals
- 16: Gelenkkopf
- 18: Pfannenschale
- 20: Pfanneneinsatz
- 22: Struktur
- 24: Aufnahmeraum
- 26: Mittelachse der Pfannenschale
- 28: Innenfläche
- 30: polseitiger Grund
- 32: Außenfläche
- 34: Berührungslinie
- 36: sphärische Lagerfläche
- 38: Rotationsachse des Pfanneneinsatzes
- 40: Femur

## Patentansprüche

1. Gelenkpfanne für eine Hüftendoprothese mit einer in den Beckenknochen (10) implantierbaren Pfannenschale (18) und einem Pfanneneinsatz (20) zur Lagerung des Gelenkkopfes (16), wobei der Pfanneneinsatz (20) mit einer sphärischen Außenfläche (32) in einem Aufnahmeraum (24) der Pfannenschale (18) sitzt,
**dadurch gekennzeichnet, dass** die Außenfläche (32) des Pfanneneinsatzes (20) die Innenfläche (28) des Aufnahmeraumes (24) in einer zur Rotationsachse (26) des Aufnahmeraumes (24) konzentrischen Berührungslinie (34) berührt, dass die Innenfläche (28) des Aufnahmeraumes (24) sich in dem Bereich dieser Berührungslinie (34) gegen den Pol des Aufnahmeraumes (24) verengt, und dass der Pfanneneinsatz (20) in dem Aufnahmeraum (24) selbsthemmend klemmbar ist.

2. Gelenkpfanne nach Anspruch 1,
**dadurch gekennzeichnet, dass** die Innenfläche (28) des Aufnahmeraumes (24) im Bereich der Berührungslinie (34) konisch ausgebildet ist.

3. Gelenkpfanne nach Anspruch 2,
**dadurch gekennzeichnet, dass** der Kegelwinkel der sich konisch verengenden Innenfläche (28) des Aufnahmeraumes (24) der Selbsthemmungswinkel der Materialpaarung von Pfannenschale (18) und Pfanneneinsatz (20) ist.

4. Gelenkpfanne nach Anspruch 3,
**dadurch gekennzeichnet, dass** der Kegelwinkel der konischen Innenfläche (28) zwischen etwa 4° und 10° liegt.

## Claims

1. Joint socket for a hip endoprosthesis having a socket shell (18) implantable in the pelvic bone (10) and a socket insert (20) as bearing for the joint head (16), the socket insert (20) being seated by a spherical outer surface (32) in a receiving space (24) of the socket shell (18),
**characterized in that** the outer surface (32) of the socket insert (20) makes contact with the inner surface (28) of the receiving space (24) along a line of contact (34) concentric with the rotational axis (26) of the receiving space (24), the inner surface (28) of the receiving space (24) narrows towards the pole of the receiving space (24) in the region of that line of contact (34), and the socket insert (20) is clampable self-lockingly in the receiving space (24).

2. Joint socket according to claim 1,
**characterized in that** the inner surface (28) of the receiving space (24) is conical in the region of the line of contact (34).

3. Joint socket according to claim 2,
**characterized in that** the cone angle of the conically narrowing inner surface (28) of the receiving space (24) is the self-locking angle of the material pairing of socket shell (18) and socket insert (20).

4. Joint socket according to claim 3,
**characterized in that** the cone angle of the conical inner surface (28) is between about 4° and 10°.

## Revendications

1. Cotyle pour une endoprothèse de la hanche comprenant une cupule (18) de cotyle pouvant être implantée dans l'os (10) du bassin et un insert (20) de cotyle destiné à loger le condyle (16), l'insert (20) de cupule à surface extérieure sphérique (32) reposant dans un espace de réception (24) de la cupule (18) de cotyle,
**caractérisé en ce que** la surface extérieure (32) de l'insert (20) de cotyle est en contact avec la surface intérieure (28) de l'espace de réception (24) selon une ligne de contact concentrique par rapport à l'axe de rotation (26) de l'espace de réception (24), la surface intérieure (28) de l'espace de réception (25) se rétrécit dans le secteur de cette ligne de contact (34) contre le pôle de l'espace de réception (24), et l'insert (20) de cotyle peut être coincé de manière autobloquante dans l'espace de réception (4).

2. Cotyle selon la revendication 1,
**caractérisé en ce que** la surface intérieure (28) de l'espace de réception (24) est formée de manière conique dans le secteur de la ligne de contact (34).

3. Cotyle selon la revendication 2,
**caractérisé en ce que** l'angle de sommet de la surface intérieure (28) se rétrécissant de manière conique de l'espace de réception (24) représente l'angle d'autoblocage de l'appariement de matériau de la cupule (18) de cotyle et de l'insert (20) de cotyle.

4. Cotyle selon la revendication 3,
**caractérisé en ce que** l'angle de sommet de la surface intérieure (28) conique est comprise entre environ 4° et 10°.
